# EUROPEAN PATENT APPLICATION

(11) **EP 0 582 477 A1**
(43) Date of publication of application: **09.02.1994**
(21) Application number: 93306204.4
(22) Date of filing: 05.08.1993
(51) Int. Cl.: G01N 33/574, C12Q 1/68, A61K 39/295

(54) **Method for diagnosing cancer and pharmaceutical composition**

(30) Priority: 06.08.1992 JP 229375/92
(71) Applicant: TOSOH CORPORATION, Yamaguchi-ken 746 (JP)
(72) Inventor: Takeshi, Azuma, Nakagyo-ku, Kyoto-shi, Kyoto (JP); Hitoshi, Hori, Fujisawa-shi, Kanagawa-ken (JP); Takao, Matsuba, Atsugi-shi, Kanagawa-ken (JP); Hiroshi, Iida, Zama-shi, Kanagawa-ken (JP); Koji, Igarashi, Kawasaki-shi, Kanagawa-ken (JP); Masayuki, Yamada, Sagamihara-shi, Kanagawa-ken (JP)
(74) Representative: Kearney, Kevin David Nicholas

(57) **Abstract**

A method for diagnosing cancer, which comprises reacting an anticathepsin E antibody or an antiprocathepsin E antibody with a sample and detecting a complex of the antibody with cathepsin E or procathepsin E in the sample.

## Description

The present invention relates to diagnosis of cancer and a pharmaceutical composition. More particularly, it relates to a method for detecting cancer by means of cathepsin E or its antibody, and a pharmaceutical product therefor.

Heretofore, as a cancer marker, of which the relation with cancer has been made clear and which has been used as an index for cancer diagnosis, there has been a substance produced by cancer cells (tumor-produced marker) such as CEA (carcinoembryonic antigen) or AFP (α-fetoprotein), or a substance produced by a biogenic body in response to formation of cancer (reactant to tumor) such as acute phase reactant. As one of conventional methods for cancer diagnosis, diagnosis of cancer has been conducted by quantifying the cancer marker in blood or in urine by EIA (enzyme immunoassay) or by RIA (radio immunoassay) or by conducting tissue staining, by means of an antibody to such a cancer marker.

Such conventional cancer markers, whether they are tumor-produced markers or reactants to tumors, are substances differing only in their quantities as between cancerous cells and non-cancerous cells, and they are not substances specifically appearing only in cancer cells. Therefore, they can not necessarily be regarded as markers specific to cancer. Accordingly, in a diagnosis employing an antibody to such a conventional cancer marker, the amount of an antigen (cancer marker substance) in blood or in urine is measured with respect to both a healthy person and a cancer patient, and the results are compared to determine a value (cut-off value) at which the healthy person and the cancer patient can be distinguished, so that if the amount of the antigen exceeding such a value is detected in blood or in urine, the possibility of cancer may be pointed out.

Thus, conventional cancer markers are substances which may be produced not only by cancer cells but also by non-cancer cells, and the determination of the amount of the antigen as the standard for cancer diagnosis is optional. For these reasons, in the conventional cancer diagnosis, a pseudo-positive or pseudo-negative result can not be avoided.

It is an object of the present invention to provide a diagnostic method employing a cancer marker specifically produced by cancer cells and a pharmaceutical compositions relating thereto.

The present inventors have discovered that no cathepsin E is produced by normal cells, and cathepsin E is produced by cancerous cells for the first time. The present invention has been accomplished on the basis of this discovery.

Namely, the present invention provides a method for diagnosing cancer, which comprises reacting an anticathepsin E antibody or an antiprocathepsin E antibody with a sample and detecting a complex of the antibody with cathepsin E or procathepsin E in the sample.

The present invention also provides a method for diagnosing cancer, which comprises reacting cathepsin E or procathepsin E as an antigen with a sample and detecting a complex of the antigen with an anticathepsin E antibody or an antiprocathepsin E antibody in the sample.

Further, the present invention provides a method for diagnosing cancer, which comprises reacting DNA or RNA capable of hybridizing with mRNA of cathepsin E, with a sample and detecting a hybridization product of DNA or RNA with mRNA of cathepsin E in the sample.

On the other hand, the present invention provides a pharmaceutical composition for diagnosing or treating cancer, which contains an anticathepsin E antibody or an antiprocathepsin E antibody as an active ingredient.

In the accompanying drawings:

Figure 1 is a view showing the results of the immunological staining of a human pancreatic cancer tissue in Example 2.

Figure 2 is a view showing the results of the immunological staining of a hamster pancreatic cancer tissue in Example 2.

Figure 3 is a view showing the results of the immunological staining of a human normal pancreatic tissue in Example 2.

Figure 4 is a view showing the results of the immunological staining of a hamster normal pancreatic tissue in Example 2.

Now, the present invention will be described in detail with reference to the preferred embodiments.

Cathepsin E is an aspartic protease which exists in gastric mucosal epithelium cells, urinary bladder cells, bone marrow cells, spleen cells, lung cells, erythrocytes and lymphocytes such as macrophage, and it is present usually in the form of a dimer consisting of two monomers with a molecular weight of 40,000 bonded by a disulfide bond. (Barrett, A.J. et al., Proteinases in Mammalian Cells and Tissues, 209-248, 1977, North-Holland, Amsterdam, Kay, J. et al., Proteases, Plenum Press, New York) . The amino acid sequences and the nucleotide sequence of cathepsin E and procathepsin E are disclosed in Azuma T. et al., THE JOURNAL OF BIOLOGICAL CHEMISTRY, Vol. 264, No. 28, pp. 16748-16753 (1989). Cathepsin E consists of 343 amino acids, and procathepsin E has 36 amino acids linked before the amino acid sequence of cathepsin E.

Cathepsin E is present only in the cell structures of normal cells and will not be secreted out of the cells (Samloff, I.M. et al., Gastroenterology, Vol. 93, p. 77-84, 1987). Its functions are not yet clearly understood, but it is believed that cathepsin E is related to the metabolic turnover of proteins in the cells and to the autoclasia of cells due to aging of the cells (Yamamoto, K. et al., J. Biochem. Vol. 105, p. 114-119, 1989).

In the pancreas, cathepsin E is not produced by normal cells and is produced for the first time by cancerous cells. Namely, this indicates that at least so far as the pancreas is concerned, cathepsin E will be a marker specific to cancer and capable of accurately diagnosing cancer. In normal cells of the liver, brain, adrenal, spermary, etc., no cathepsin has been detected (Hideaki Sakai, et al., Biochimica et Biophysica Acta., Vol. 991, p. 367-375, 1989), but it is conceivable that also in these organs, cathepsin E is produced by cancerous cells. Because, the phenomenon that cathepsin E is produced specifically by the cancerous cells of the pancreas, is assumed to be derived from a change in the metabolism within the cells or from a change in the control of gene expression due to the change of normal cells to cancerous cells. At least a part of such changes is considered to occur commonly to cancerous cells. Accordingly, it is possible to diagnose cancer by detecting cathepsin E.

Further, with respect to certain cancer cells, a phenomenon has been observed such that localization of cathepsin E in the cells changes (T. Saku et al., Gut., Vol. 31, p. 1250-1255, 1990). This indicates that the transport route of the protein synthesized in the cells has changed due to transformation of cells. Thus, it is considered that cathepsin E which is usually present only in the cells in the case of normal cells, is secreted out of the cells in the case of cancer cells. In such a case, an anticathepsin E antibody may appear in the biogenic body. Thus, it is possible to diagnose cancer by detecting the anticathepsin E antibody.

Human cathepsin E may be obtained, for example, by extracting cathepsin E from e.g. the above-mentioned normal cells. Otherwise, human cathepsin E or prohuman cathepsin E may be recovered by purification from the supernatant of culture of e.g. Escherichia coli, yeast or animal cells transformed by cathepsin E expression plasmids to produce cathepsin E, or from the cultured cells. Of course, it may not be necessary to use the human cathepsin E protein in its entirety as the antigen. Namely, a part of the amino acid sequence thereof may be artificially synthesized in the form of a peptide, which may be used as the antigen, or a fragmented cathepsin E may also be employed.

As the antibody to cathepsin E, a monoclonal antibody or a polyclonal antibody may be used in the present invention. The polyclonal antibody may be obtained from a serum of an animal such as a rabbit by immunizing the animal using purified human cathepsin E as the antigen. The monoclonal antibody can be obtained by a conventional method, or it may be obtained by recently developed antibody gene engineering.

In the present invention, cathepsin E in a sample can be detected by reacting an antibody thereto and detecting a complex as the reaction product. To facilitate the detection, it is advisable that a label such as an enzyme, a radioactive substance or a fluorescent substance is preliminarily bonded to the antibody. Inversely, an anticathepsin E antibody in a sample may be detected by reacting cathepsin E thereto and detecting a complex as the reaction product. Also in this case, it is advisable that a label such as an enzyme, a radioactive substance or a fluorescent substance is preliminarily bonded to cathepsin E.

As the sample to be measured, it is possible to employ fragments of tissues of various organs, blood serum, urine, pancreatic juice, gastric juice or other biogenic liquid components. When a fragment of tissue is used as the sample, the tissue fragment of an organ prepared in accordance with a conventional method, is subjected to immunological staining by means of an anticathepsin E antibody, whereupon formation of cancer will be judged by observing the presence or absence of the expression of cathepsin E.

In a case where the sample is a biogenic liquid component, it is possible to diagnose cancer by detecting cathepsin E by means of an anticathepsin E antibody by a conventional measuring method such as a sandwich EIA method, a competition method, an agglutination reaction method or a western blotting method. Further, when cathepsin E which is normally not secreted out of cells, is secreted from cancerous cells, an anticathepsin E antibody will be formed in the body. In such a case, it is possible to diagnose cancer by detecting an anticathepsin E antibody by means of cathepsin E by a conventional measuring method such as a sandwich method, a competition method, an agglutination reaction method or a western blotting method.

Further, in the present invention, for detecting cathepsin E for the diagnosis of cancer, it is possible to employ not only a method wherein cathepsin E is detected as a protein, but also a method wherein mRNA of cathepsin E is detected. Namely, a sample is reacted with DNA or RNA hybridizable with mRNA of cathepsin E, whereupon a hybridization product of DNA or RNA with mRNA of cathepsin E in the sample, may be detected. As the method for detecting mRNA, an in situ hybridization method, a northern blotting method or a PCR method may, for example, be mentioned.

The present invention also provides a pharmaceutical composition for diagnosing or treating cancer, which contains an anticathepsin E antibody or an antiprocathepsin E antibody as an active ingredient. As the pharmaceutical composition for diagnosing cancer, a measurement kit for e.g. a sandwich EIA method, a competition method or an agglutination reaction method, may, for example, be mentioned. As the pharmaceutical composition for treating cancer, a composition for missile treatment having a toxin or a drug bonded to the antibody, may, for example, be mentioned.

In the foregoing, the present invention has been described with respect to cathepsin E. However, procathepsin E as its precursor can also be used in the same manner as cathepsin E for the diagnostic method of the present invention as well as for the pharmaceutical composition for diagnosing or treating cancer of the present invention.

The present invention has made the relation clear between cancer and cathepsin E or procathepsin E, the relation of which with cancer has not been known before, and it is capable of presenting a new method for diagnosing cancer. Further, it is capable of presenting a new pharmaceutical composition for diagnosing or treating cancer, based on the relation with cancer.

Now, the present invention will be described in further detail with reference to Examples. However, it should be understood that the present invention is by no means restricted by such specific Examples.

### EXAMPLE 1

### Induction of experimental cancer employing hamster

A method for inducing cancer experimentally in the pancreas of a hamster is disclosed in detail by Parviz Pour et al., J. NATL. CANCER INST., Vol. 58, No. 5, p. 1449-1453, 1977 and by Osamu Ishikawa et al., Nisshogai Kaishi, Vol. 20, No. 9, p. 2147-2152, 1987. In accordance with such a method, experimental cancer was produced in the pancreas of a hamster.

Namely, to a golden hamster of 8 weeks old, BOP (N-nitrosobis (2-oxopropyl)amine, dissolved in 0.5 ml of a 0.9% sodium chloride aqueous solution was administered by hypodermic injection once a week in an amount of 5 mg per kg of the body weight. By this method, pancreatic cancer was developed at a high rate of about 80% after 30 to 40 weeks from the initiation of the administration of BOP.

### EXAMPLE 2

### Immunological staining of the pancreatic cancer tissue by means of an anticathepsin E blood serum and the results thereof

The anticathepsin E blood serum was obtained by immunizing a rabbit with a purified standard product of cathepsin E in accordance with a conventional method, as disclosed by Saku T. et al., J. Biochem. (Tokyo) 110, 956-964 (1991).

The preparation and purification of the standard product of cathepsin E were conducted in accordance with Yamamoto K. et al., Biochem. Biophys. Acta 790, 208-228 (1984).

Immunological staining of human pancreatic cancer, normal pancreas of human, normal pancreas of a hamster and the pancreatic cancer induced in Example 1, was conducted by means of Hystfine SAB-PO (R) kit (manufactured by Nichirei) in accordance with the method described in the attachment to the kit. Namely, the tissues obtained in accordance with a conventional method from the normal and cancerous human pancreas or hamster pancreas, respectively, were fixed with a 10% buffer formalin. The fixed tissues were embedded in paraffin and sectioned to obtain sections of from 4 to 6 µm, which were fixed on slide glass with 0.02% poly-L-lysin.

The slide glass thus prepared was subjected to paraffin removal treatment and then treated with blocking reagents I and II. For the treatment with the blocking reagent I, the operation method mentions about treatment with a 3% hydrogen peroxide aqueous solution, but in this Example, a hydrogen peroxide aqueous solution diluted to 0.3% with methanol was used instead of the 3% hydrogen peroxide aqueous solution. The reactive component contained in blocking reagent II is 10% goat normal serum.

In addition to such reactive components, the blocking reagents may contain additives (such as preservatives). These blocking agents are used to prevent extra reactions other than the detection reaction.

After the above operation, an anticathepsin E rabbit blood serum diluted 1,000 times with a 0.01M phosphate buffer physiological saline solution as the first antibody and a biotin-labeled antirabbit IgG antibody as the second antibody, were reacted to the tissue sections fixed to the slide glass. After the reaction with the first and second antibodies, a peroxidase-labeled streptoabidin was added and reacted, and a color developing agent constituting a substrate for peroxidase was added and further reacted, to develop a color showing the presence of a peroxidase, and the developed color was detected.

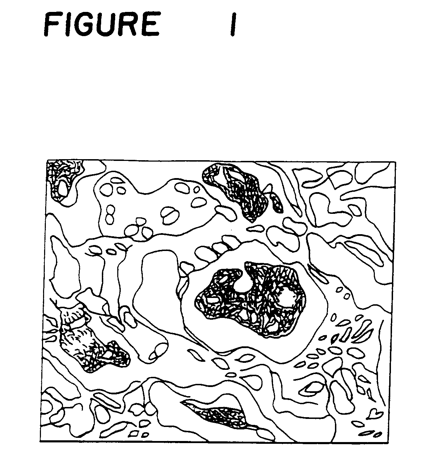

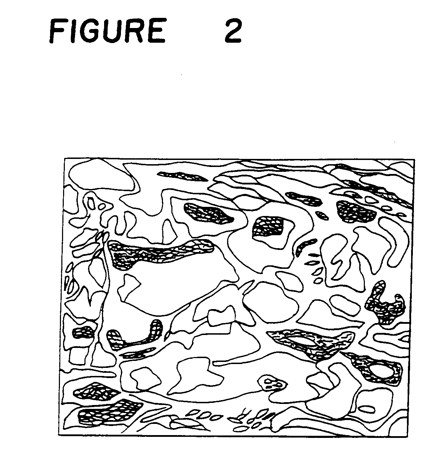

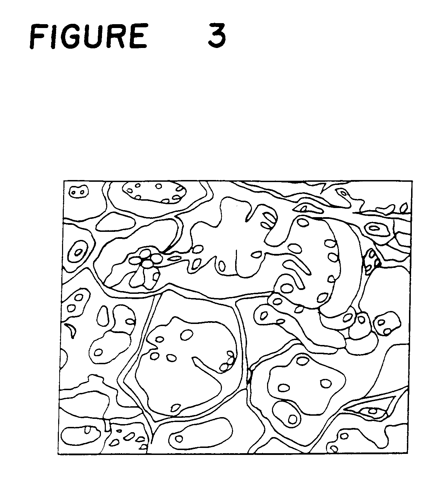

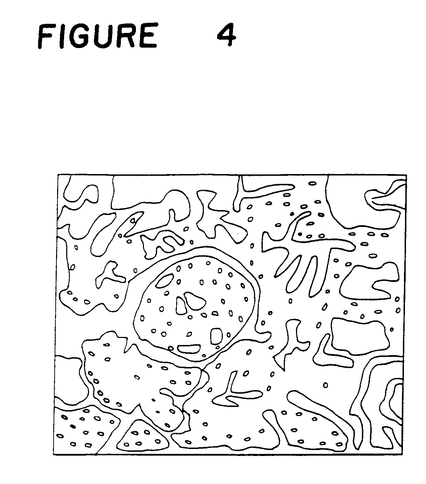

After the above operation, a positive reaction was observed by an optical microscope. With the human normal pancreatic tissue in Figure 3 and with the normal pancreatic tissue of the hamster in Figure 4, the tissues were found to be not stained, as is evident from the drawings. However, in Figures 1 and 2, the pancreatic cancer tissues of human and hamster, respectively, were found to be stained. Thus, it is evident that in human and hamster, no production of cathepsin E is observed in normal pancreatic cells, and cathepsin E is produced only in the cancer cells, whereby diagnosis of cancer can be conducted.

## Claims

1. A method for diagnosing cancer, which comprises reacting an antibody to cathepsin E or procathepsin E with a sample and detecting a complex of the antibody with cathepsin E or procathepsin E in the sample, the presence of such complex being indicative of the presence of cancer.

2. A method for diagnosing cancer, which comprises reacting cathepsin E or procathepsin E as an antigen with a sample and detecting a complex of the antigen with an antibody to cathepsin E or procathepsin E in the sample, the presence of such complex being indicative of the presence of cancer.

3. A method for diagnosing cancer, which comprises reacting DNA or RNA capable of hybridizing with mRNA of cathepsin E, with a sample and detecting a hybridization product of DNA or RNA with mRNA of cathepsin E in the sample, the presence of such hybridization product being indicative of the presence of cancer.

4. A composition for diagnosing cancer, characterised in that it comprises an anticathepsin E antibody or an antiprocathepsin E antibody as an active ingredient.

5. A pharmaceutical composition for treating cancer characterised in that it comprises an anticathepsin E antibody or an antiprocathepsin E antibody as an active ingredient.
